Europäisches Patentamt

European Patent Office

Office européen des brevets

(11) Publication number: **0 038 013**
**B1**

(12) **EUROPEAN PATENT SPECIFICATION**

(45) Date of publication of patent specification: **10.07.85**

(51) Int. Cl.⁴: **A 61 K 9/00**

(21) Application number: **81102618.6**

(22) Date of filing: **07.04.81**

(54) Injectable oxytetracycline compositions.

(30) Priority: **10.04.80 US 139147**

(43) Date of publication of application:
**21.10.81 Bulletin 81/42**

(45) Publication of the grant of the patent:
**10.07.85 Bulletin 85/28**

(84) Designated Contracting States:
**AT BE CH DE FR GB IT LI LU NL SE**

(56) References cited:
**FR-A-2 081 434**
**FR-A-2 258 187**
**GB-A-1 250 304**
**US-A-3 004 894**
**US-A-4 018 889**

(73) Proprietor: **SDS BIOTECH CORPORATION**
**P.O. Box 348 7528 Auburn Road**
**Painesville Ohio 44077 (US)**

(72) Inventor: **Hacke, Walter**
**75 Sunderland Avenue**
**Rutherford New Jersey (US)**
Inventor: **Horn, Herman**
**165 Fields Avenue**
**Staten Island New York (US)**

(74) Representative: **Huber, Bernhard, Dipl.-Chem.
et al**
**Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

The file contains technical information
submitted after the application was filed and
not included in this specification

Courier Press, Leamington Spa, England.

# 0 038 013

**Description**

1. Field of the invention
   This invention relates to oxytetracycline injectable compositions having increased antibiotic content.

2. Description of the prior art
   Heretofore, preparations of oxytetracycline compositions suitable for injection have suffered from relatively high viscosities, poor stability and limited concentration of antibiotic. Such preparations have employed solvents such as propylene glycol, glycerol and polyethylene glycols as well as their mixtures with ethanol. High viscosities are particularly noticeable when injectable compositions containing polyhydric alcohols as solvents are used at low temperatures. Such high viscosities are often encountered when the compositions are used at the cool temperatures prevailing in barns, open feedlots or pastures. High viscosity compositions are objectionable because aspiration of a viscous composition into a hypodermic syringe and subsequent injection of the composition are both difficult and slow. Further, high viscosities also increase the time required to inject a large herd of animals. Other solvents may cause tissue irritation which is particularly undesirable in an animal as they may cause localized concentration of the antibiotic in the tissue and render the affected area undesirable for human consumption.
   It is known according to United States Patent US—A—3,712,949—Greenbaum et al—January 23, 1973, (equivalent to GB—A—1 250 304) to prepare injectable oxytetracycline solutions utilizing glycerol formal as the solvent, a water soluble magnesium salt such as magnesium chloride hexahydrate, antioxidant, buffering agent and water. These compositions overcome the disadvantages specified above, i.e., they are characterized as having lower viscosities at low temperatures and having excellent stability as to color, antibiotic potency and physical state. However, the maximum antibiotic concentration which has been achieved is approximately 15% by weight of the total composition. It is desirable to prepare compositions having greater antibiotic concentration. This is of great importance because a composition having a greater antibiotic concentration permits reduction of the number of injections at any one time in large animals such as cattle. In the case of a large herd, this reduces both time and labor. Additionally, a reduced number of injection sites in an animal brings about less damage to the quality of the meat.

Summary of the invention
   Oxytetracycline injectable compositions having increased antibiotic concentrations where glycerol formal is the solvent have been achieved by employing particular magnesium compounds, viz., magnesium oxide, magnesium hydroxide, magnesium carbonate, magnesium acetate and with oxytetracycline base only, magnesium chloride. These compositions can contain from 15% by weight to 35% by weight oxytetracycline. These compositions permit a reduction in the number of injections at any one time in large animals. Further, they are characterized by their stability and low viscosities at low temperatures. Thus, fluid oxytetracycline compositions of enhanced antibiotic content suitable for injection over a wide range of temperatures, including low temperatures are provided for. Excellent stability of color, potency and physical state is achieved. Further, animals injected with these compositions are free of irritation at the site of the injection.

Description of the preferred embodiments
   The relative proportions of the constituents can be varied widely. For example, the quantity of antibiotic such as oxytetracycline base or an acid addition salt thereof can vary from 15.0 to 35.0 parts by weight.
   The mole ratio of magnesium compound such as magnesium oxide to oxytetracycline base or acid addition salt can be varied from 0.8 to 1.2 moles of magnesium compound per mole of antibiotic. Preferably the mole ratio of magnesium compound to oxytetracycline base is about 1:1 while the mole ratio of magnesium compound to oxytetracycline hydrochloride is about 0.8 to 1. Lower ratios tend to give deeper color on standing while higher ratios tend to give both deeper color and cause precipitation on standing. With higher quantities of antibiotic, the quantity of magnesium compound should be maintained near its lower range, i.e., the mole ratio of magnesium compound to antibiotic should be 0.8 to 1 to avoid higher concentrations of magnesium compound which may adversely affect the viscosity.

   The quantity of glycerol formal can vary from 50 to 95 parts by weight, the upper limit being regulated, of course, by the quantities of the other constituents.
   The quantity of water can be varied from 10 to as much as 45 parts by weight. Above 45 parts by weight of water, a turbid composition is formed. The presence of water is desirable to assist solution of the inorganics and to obtain an injectable composition which causes no visible tissue damage. Ethyl alcohol up to 20 parts by weight can be substituted for part of the water or part of the glycerol formal in the composition to obtain a composition having even a lower viscosity.

   An effective amount of an antioxidant is added. Sufficient buffering agent is added to provide a composition having a pH of from 6 to 9.5.
   Glycerol formal, a condensation product of glycerol and formaldehyde is obtained as a mixture of 4-hydroxymethyl-1,3-dioxolane and 5-hydroxy-1,3-dioxane. The mixture obtained as the product of the

2

# 0 038 013

reaction, or a mixture made by fortifying the product of the reaction with one of the components, or the individual components can be used in the present invention.

With respect to the magnesium compound, magnesium oxide, magnesium hydroxide, magnesium carbonate and magnesium acetate can be used. Magnesium oxide is preferred. With oxytetracycline base only magnesium chloride is used. When the magnesium compound is selected from magnesium chloride and magnesium acetate, then the antibiotic is present in an amount greater than 15 and up to 35 parts by weight.

Antioxidants are used in addition to manufacturing and storing the compositions of this invention in an inert atmosphere. The antioxidants assist in the stabilization of the color and potency of the compositions. Any antioxidant which is physiologically acceptable for use in a parenteral drug composition and which is compatible with oxytetracycline can be used in the present invention. Suitable antioxidants include sodium bisulfite, sodium metabisulfite, sodium formaldehydesulfoxylate and monothioglycerine. Generally from 0.05 to 5 parts by weight, preferably 0.5 to 2 parts by weight of antioxidant are used.

An inert atmosphere such as nitrogen or helium is not necessary for maintenance of potency, but aids considerably in the retention of a light colored composition on long storage.

The compositions of the present invention are prepared by mixing an oxytetracycline antibiotic, in its free base form (oxytetracycline base) or as an acid addition salt, with the magnesium compound in water or the glycerol formal solvent. When water is employed, the glycerol formal solvent is then added to the aqueous solution in appropriate quantity.

The pH of the composition is then adjusted to 6.0 to 9.5 with a buffering agent, e.g., physiologically acceptable bases such as sodium hydroxide, potassium carbonate, ammonia or physiologically acceptable lower aliphatic primary, secondary and tertiary amines having up to about six carbon atoms per group attached to the amino nitrogen atom. These amines include ethanolamine (2-aminoethanol), diethylamine, ethylamine, triethanolamine, diethanolamine, arginine and glucosamine. The final pH of the composition is ordinarily not critical, however, best stability appears to reside with moderate pH value, i.e., between 6.0 to 9.5. Lower pH values cause too rapid decomposition of the antioxidant while too high a pH increases color formation. Also when the final pH is above 9.5, physiological compatibility of the composition with the muscle tissue is decreased. A final pH slightly on the alkaline side is preferred in order to minimize local tissue irritation on parenteral administration. The most satisfactory range is from 6.0 to 9.5, preferably from 7.5 to 8.5.

For injection, the finished composition must be sterile. That is, sterile components and sterile· conditions of manufacture must be employed, or alternatively, the composition itself must be sterilized after manufacture such as by sterile filtration.

Regarding the selection of the antibiotic, oxytetracycline free base or an acid addition salt of same such as oxytetracycline hydrochloride, oxytetracycline phosphate, oxytetracycline sulfate or oxytetracycline acetate can be used. Where a light colored final product is desired, a light colored antibiotic should be used.

For a fuller understanding of this invention, reference may be made to the following examples. These examples are merely to illustrate the invention and are not to be construed in a limiting sense.

In the following examples, the glycerol formal contained 50% by weight of 4-hydroxymethyl-1,3-dioxolane and 50% by weight of 5-hydroxy-1,3-dioxane. This material can be prepared as described in Example I of United States Patent No. 3,712,949.

The oxytetracycline assay procedure utilized is described in 21 Code of Federal Regulations Part 446.265, page 541 and Part 446.267, page 542, April 1, 1978 revision. See portions entitled Oxytetracycline injectable and references referred to therein.

Example I

This example describes the preparation and stability of a 20% by weight oxytetracycline solution using glycerol formal as the solvent and a water soluble magnesium salt.

Under nitrogen atmosphere, 57.5 grams oxytetracycline hydrochloride having a potency of 890 mcg/mg were dissolved in a solution of 30 ml distilled water and 48 grams of glycerol formal. The resulting mixture was stirred until dispersed. Then 18.0 grams of reagent grade magnesium chloride hexahydrate was added. The mole ratio of magnesium to oxytetracycline was 0.8 to 1. Additional glycerol formal was added so that the total amount of glycerol formal used was 195 grams. The resulting mixture was warmed to 40°C and stirred until a clear solution was obtained (about 1 1/4 hrs.). The resulting solution was cooled to about 20—25°C and sufficient monoethanolamine was added over about 1 hour to adjust the pH to 8.3—8.4. Then a solution of 2.5 grams of sodium formaldehyde sulfoxylate in 6 ml water was slowly added (a slight yellow precipitate formed which dissolved after a few minutes) and the resulting solution stirred until a constant pH of 8.3—8.4 was attained. Additional monoethanolamine was added to maintain the pH. About 2 hours were required during which time a total of 23.1 ml of monoethanolamine was added. The potency of the resulting solution was 210 mg oxytetracycline/ml. Its viscosity was 140 centipoise at 5°C and 90 centipoise at 21°C. This solution was filtered through a 0.2 μm membrane filter.

Stability studies were conducted. Samples on storage at 45°C were stable only for 29 to 39 days while samples on storage at 37°C were stable only for 137 to 153 days. Formation of a yellow precipitate was taken as evidence of instability.

3

Example II

This example describes the preparation and stability of a 20% by weight oxytetracycline base solution.

Under nitrogen atmosphere, 2.0 grams of sodium formaldehyde sulfoxylate were dissolved in 50 ml distilled water. Then 3.8 grams magnesium oxide were added, the mixture stirred and 46 grams oxytetracycline base having a potency of 920 mcg/mg and 144 grams glycerol formal were added. The mole ratio of magnesium to oxytetracycline was 1 to 1. The resulting mixture was heated at 40°C for 20 minutes until a practically clear solution was obtained. It was cooled to 20°C and the pH adjusted to 8.4 with 1.4 ml monoethanolamine. About 2 hours was required for pH stabilization. The solution had a potency of 206 mg oxytetracycline/ml. The solution containing 20.6% by weight oxytetracycline was filtered through a 0.2 µm membrane filter.

Samples were stored at 37°C for over 200 days. No yellow precipitation was observed.

Additional preparations containing oxytetracycline base tabulated below, were prepared in a manner similar to the preparation of the composition of Example II.

| Example No. | Parts by weight | | | |
|---|---|---|---|---|
| | III | IV | V | VI |
| Ingredients: | | | | |
| Oxytetracycline base (potency: 920 mcg/mg) | 22.4 | 48.0 | 48.0 | 48.0 |
| Magnesium oxide | 1.9 | 4.0 | 4.0 | 4.6 |
| 2-aminoethanol | 0.8 | 1.1 | 1.0 | 0 |
| Sodium formaldehyde sulfoxylate | 1.0 | 2.1 | 2.0 | 2.0 |
| Glycerol formal | 72.0 | 144.0 | 130.0 | 130.0 |
| Water | 20.0 | 60.0 | 72.0 | 72.0 |
| % Oxytetracycline | 20.2 | 19.5 | 20.3 | 20.4 |
| Mole ratio of magnesium to oxytetracycline | 1.1 to 1 | 1 to 1 | 1 to 1 | 1.2 to 1 |

Example VII

This Example describes the preparation of a 20% by weight oxytetracycline solution prepared from oxytetracycline base.

A 2 liter glass vessel was evacuated and flushed with nitrogen. Then 576 ml of distilled water was added, the vessel stirred and 9.6 grams of sodium formaldehyde sulfoxylate added. Eight minutes were required to dissolve the antioxidant. Glycerol formal, 814 ml, was added. Magnesium oxide 30.5 grams and oxytetracycline base of 91% purity, 384 grams, were added. The antibiotic was rinsed in with an additional 50 ml of glycerol formal. Approximately 20 minutes was required for addition of magnesium oxide and oxytetracycline; seven minutes for dissolving the reactants. During addition of the oxytetracycline the temperature rose to 36°C. Occasional heating to dissolve the oxytetracycline was required. Stirring was continued for one-half hour, the temperature at 30°C. The pH was 7.4. Monoethanolamine, 11.8 ml, was added dropwise during the course of eight minutes with stirring. The pH, after addition, was 8.2. Stirring was continued and the system allowed to equilibrate for two hours during which an additional 1.6 ml of monoethanolamine were added. Final pH was 8.2; final volume was 1,700 ml.

The solution was pre-filtered through No. 3 Whatman filter paper. The solution was then filtered through a 0.2 µm membrane filter (Millipore Corporation, Bedford, Mass.).

The solution had the following characteristics:

| | |
|---|---|
| Antibiotic potency (calculated) | 206 mg/ml |
| Antibiotic potency (by duplicate assay) | 200, 194 mg/ml |
| Specific gravity | 1.192 |
| Viscosity | 19.3 mPas at 24°C; 22.1 mPas at 22°C and 50 mPas at 0°C |
| Mole ratio of magnesium to antibiotic | 1 to 1 |

Stability studies were conducted both at ambient temperature and at 37°C with the data reported below. Assay data is the average of two determinations.

| Time (months) | Initial | 3 | 6 | 9 | 12 |
|---|---|---|---|---|---|
| | | Ambient temperature | | | |
| Antibiotic potency (mg/ml) | 197 | 208 | 201 | 207 | 207 |
| Color | Amber | Amber | Amber | Amber | Dark Amber |
| pH | 8.2 | 8.1 | 8.1 | 8.2 | 8.3 |
| | | | At 37°C | | |
| Antibiotic potency (mg/ml) | 197 | 208 | 197 | 196 | 203 |
| Color | Amber | Amber | Dark Amber | Dark Amber | Dark Amber |
| pH | 8.2 | 8.2 | 8.2 | 8.3 | 8.4 |

Example VIII

This Example describes the preparation of a 20% by weight oxytetracycline solution prepared from oxytetracycline hydrochloride.

A 2 liter glass vessel was evacuated and flushed with nitrogen. Then 300 ml of water and 23 grams of magnesium oxide were added with stirring to disperse the magnesium oxide. Glycerol formal, 800 ml and oxytetracycline hydrochloride of 91% oxytetracycline purity, 360 grams, were added with stirring to dissolve the antibiotic. Antibiotic addition required ten minutes during which time, the temperature rose to 41°C. Glycerol formal, 130 ml, was added to rinse in the antibiotic followed by stirring for one hour. The temperature was 32°C and pH was 3.8. Monoethanolamine, 80 ml was added over 40 minutes. The temperature was 32°C and pH rose to 8.2. A sodium formaldehyde sulfoxylate solution, 8 grams dissolved in 20 ml of water, was added. A precipitate was formed which dissolved within five minutes. Stirring was carried out one and one-half hours to allow for pH equilibrium. The pH was then adjusted to 8.2—8.4 with additional monoethanolamine. Final volume of the solution was 1,510 ml.

The solution was filtered through a 0.2 micron membrane filter.

The solution had the following characteristics:

| | |
|---|---|
| Antibiotic potency (calculated) | 206 mg/ml |
| Antibiotic potency (by duplicate assay) | 193, 202 mg/ml |
| Specific gravity | 1.233 |
| Viscosity | 47.3 mPas at 23°C |
| Mole ratio of magnesium to antibiotic | 0.8 to 1 |

Stability studies were conducted both at ambient temperature and at 37°C with the data reported below. Assay data is the average of two determinations.

| Time (months) | Initial | 3 | 6 | 9 | 12 |
|---|---|---|---|---|---|

**Ambient temperature**

| | | | | | |
|---|---|---|---|---|---|
| Antibiotic potency (mg/ml) | 198 | 212 | 212 | 206 | 206 |
| Color | Light Amber | Light Amber | Light Amber | Light Amber | Amber |
| pH | 8.6 | 8.5 | 8.3 | 8.5 | 8.7 |

**At 37°C**

| | | | | | |
|---|---|---|---|---|---|
| Antibiotic potency (mg/ml) | 198 | 215 | 189 | 203 | 206 |
| Color | Light Amber | Light Amber | Light Amber | Light Amber | Dark Amber |
| pH | 8.6 | 8.6 | 8.3 | 8.5 | 8.7 |

The following oxytetracycline solutions were prepared from oxytetracycline base using the procedure of Example VII.

| Example No. | IX | X | XI | XII | XIII | XIV |
|---|---|---|---|---|---|---|
| Ingredients: | | | | | | |
| Distilled water (ml) | 90 | 48 | 70 | 66 | 38 | 70 |
| Glycerol formal (ml) | 90 | 72 | 105 | 100 | 38 | 105 |
| Magnesium acetate tetrahydrate (grams) | — | — | — | — | — | 19.4 |
| Magnesium oxide (grams) | 4.0 | — | — | 4.75 | 2.7 | — |
| Basic magnesium carbonate (grams) | — | 11 | — | — | — | — |
| Magnesium hydroxide (grams) | — | — | 5.7 | — | — | — |
| Oxytetracycline base potency 920 mcg/mg (grams) | 48 | 37 | 48 | 60.0* | 34 | 49* |
| Sodium formaldehyde sulfoxylate (grams) | 2.1 | 1.9 | 2.0 | 1.2 | 1.0 | 1.1 |
| Monoethanolamine (ml) | — | 1.0 | 2.0 | 1.5 | 1.0 | 13.25 |
| pH | 8.5 | 8.3 | 8.4 | 8.5 | 8.4 | 8.3 |
| Antibiotic potency by assay (mg/ml) | 202 | 198 | 200 | 250—260 | 318 | 203 |
| Mole ratio of magnesium to antibiotic | 1 to 1 | 1.2 to 1 | 1 to 1 | 1 to 1 | 1 to 1 | 1 to 1 |
| % by wt. of antibiotic | 20 | 20 | 20 | 25 | 32 | 20 |

*Oxytetracycline potency was 912/mcg/mg

**0 038 013**

Example XV

This Example describes tests conducted in pigs to evaluate blood levels of antibiotic, local reaction at the site of injection and tissue residues of antibiotic.

Two formulas were utilized, each containing approximately 200 mg oxytetracycline/ml (approximately 20% by weight oxytetracycline). The composition of each formula is set forth below.

| Formula No. | A | B |
|---|---|---|
| Ingredients: | | |
| Distilled water (ml) | 150 | 192 |
| Glycerol formal (ml) | 450 | 288 |
| Magnesium oxide (grams) | 11.5 | 10.2 |
| Oxytetracycline hydrochloride, potency 910 mcg/mg (grams) | 180 | — |
| Oxytetracycline base, potency 910 mcg/mg (grams) | — | 128 |
| Monoethanolamine (ml) | 37.8 | 4.45 |
| Sodium formaldehyde sulfoxylate (grams) | 4.5 | 3.2 |
| pH | 8.2 | 8.2 |
| Antibiotic potency by assay (mg/ml) | 202 | 203 |
| Mole ratio of magnesium to antibiotic | 0.85 to 1 | 1.1 to 1 |
| % by wt. antibiotic | 20 | 20 |

Eight healthy pigs about three months old, were ear tagged with numbers at the time of purchase. These pigs weighing 21.8—30.9 kg were kept in a 4.9 m×8.5 m (16'×28') covered concrete floor pen bedded with wood shavings. All pigs were fed antibiotic free grower ration containing 15% of protein. Water was supplied free choice.

The experimental design is shown in Table I below.

TABLE I

| Formula | No. of Pigs | Dose of injectable intramuscular | Serum sampling post injection | Slaughter day post injection |
|---|---|---|---|---|
| Control | 2 | Control (no injection given) | 0, 2, 4, 8, 12, and 24 hours post injection and every 24 hours for next 6 days | One pig each on day 20 and 30 |
| A | 3 | 1 ml/10 kg of body weight (20 mg of OTC/ kg of body weight) | Same as above | One pig each on day 25 and 30 |
| B | 3 | Same as above | Same as above | Same as above |

The pigs weighed 28.1—30.9/kg after 20 days of acclimation period. Blood samples were collected from all pigs before injection. The two control pigs were not injected with any oxytetracycline formulation. The respective formulations of oxytetracycline were injected intramuscularly at doses of 20 mg of

7

**0 038 013**

oxytetracycline per kg body weight. Blood samples were collected from all pigs at 2, 4, 8, 12 and 24 hours post injection and at every 24 hours during next 6 days. Clear serum was collected from all blood samples and kept frozen. Oxytetracycline assays were conducted by method recognized by Food and Drug Administration. See Kramer, J., et al, "Antibiotic Residues in Milk, Dairy Products, and Animal Tissues: Methods, Reports, and Protocols". National Center for Antibiotic Analysis, Food and Drug Administration, Washington, D.C. 1968. One pig administered Formula A died soon after 8 hour bleeding because of a rip in the vena cava. The average value of oxytetracycline serum level for each injection group is set forth in Table II below.

TABLE II
Average oxytetracycline serum levels after intramuscular injection of oxytetracyline
20% formulations in pigs

| Oxytetracycline injectable formula | Hours post injection/mg of oxytetracycline/gram of serum | | | | | | | | | | | |
|---|---|---|---|---|---|---|---|---|---|---|---|---|
| | 0 | 2 | 4 | 8 | 12 | 24 | 48 | 72 | 96 | 120 | 144 | 168 |
| A | <0.07 | 2.25 | 2.73 | 2.35 | 1.94 | 1.13 | 0.41 | 0.22 | 0.16 | 0.08 | 0.07 | 0.07 |
| B | <0.08 | 4.05 | 3.74 | 3.11 | 2.69 | 1.47 | 0.41 | 0.26 | 0.21 | 0.10 | <0.08 | <0.08 |
| Control | <0.04 | <0.04 | <0.04 | <0.04 | <0.04 | <0.04 | <0.04 | <0.04 | <0.04 | <0.04 | <0.04 | <0.04 |

Results indicate that Formula B gave sustained higher serum levels of oxytetracycline up to 5 days post injection when compared to the data on Formula A.

All injection sites were examined for redness, swelling, hardness, abscess, sloughing or any other local reaction before the animals were sacrificed. No unusual local reaction was observed at the injection sites. All injection sites were incised after sacrifice of the pigs. None of the injection sites showed reaction to be a problem in clinical use of these formulations.

Muscle from the injection sites, samples of liver, kidney, fat and muscle from non-injected area were assayed for oxytetracycline residues using FDA recognized method. See Kramer, J., et al, "Antibiotic Residues in Milk Dairy Products, and Animal Tissue: Methods, Reports and Protocols". National Center for Antibiotic Analysis, Food and Drug Administration, Washington, D.C. 1968. The results of tissue assays are reported in Table III below.

TABLE III

Tissue residues of oxytetracycline after intramuscular injection of 20% oxytetracycline injectables at a dose of 20 mg/kg body weight

| Formula | No. of Pigs slaughtered on day post injection | | | Tissue residues of oxytetracycline mg/g | | | | |
|---------|----|----|----|--------|--------|-------|------|----------------|
| | 20 | 25 | 30 | Muscle | Kidney | Liver | Fat | Injection site |
| A | | 1 | | <0.02 | 0.22 | 0.94 | 0.12 | 0.14 |
| | | | 1 | 0.04 | 0.16 | <0.06 | 0.10 | 0.08 |
| B | 1 | | | 0.39 | 0.18 | <0.06 | 0.04 | <0.06 |
| | | 1 | | 0.14 | 0.17 | <0.06 | <0.04 | <0.06 |
| | | | 1 | <0.02 | 0.17 | <0.06 | <0.04 | <0.06 |

The data indicates that pigs sacrificed on day 20, 25, and 30 post injection with Formula B showed comparatively lower residues than pigs injected with Formula A on corresponding days.

Example XVI

This Example describes the preparation of a 20% by weight oxytetracycline solution prepared from oxytetracycline hydrochloride.

Distilled water (38 ml), 13.3 grams magnesium acetate tetrahydrate, 45 grams oxytetracycline hydrochloride having a potency of 910 mcg/mg and 115 ml glycerol formal were stirred under nitrogen atmosphere at 30—40°C for about one hour. The clear solution was allowed to cool to ambient temperature, the pH adjusted to about 8—8.2 with 15 ml monoethanolamine and 1.0 gram sodium formaldehyde sulfoxylate then added. After about one hour, the sodium formaldehyde sulfoxylate had dissolved and stirring was continued for about two hours to a constant pH of 8.3, during which time monoethanolamine was added as required. The total volume used was 16.6 ml. The volume of oxytetracycline solution was 205 ml and it assayed 208 mg/ml oxytetracycline (duplicate assays). The mole ratio of magnesium to antibiotic was 0.8 to 1. After 30 days of storage at 45°C, and 37°C and at room temperature, no oxytetracycline precipitate was observed.

Example XVII

This Example describes the preparation of a 20% by weight oxytetracycline solution prepared from oxytetracycline base.

The procedure was the same as Example VIII, but using the following:

| Ingredients | Quantity |
|-------------|----------|
| Distilled water | 38 ml |
| Magnesium chloride hexahydrate | 16.8 grams |
| Oxytetracycline (potency: 905 mcg/mg) | 45 grams |
| Glycerol formal | 115 ml |
| Monoethanolamine | 13.2 ml |
| Sodium formaldehyde sulfoxylate | 1.0 gram |

The volume of antibiotic solution was 200 ml. Final pH was 8.25 and assayed 200 mg/ml oxytetracycline (duplicate assays). Mole ratio of magnesium to antibiotic was 1 to 1.

After 30 days of storage at 45°C and 37°C, no oxytetracycline precipitate was observed. At room temperature, no oxytetra-precipitate was observed even after two months.

While the invention has been described with reference to certain specific embodiments thereof, it is understood that it is not to be so limited since alterations and changes may be made therein which are within the full intended scope of the appended claims.

## Claims for the Contracting States: BE CH DE FR GB IT LI LU NL SE

1. A stable, injectable antibiotic composition of increased antibiotic concentration for parenteral administration comprising:

(a) from 15 to 35 parts by weight of an antibiotic selected from the group consisting of oxytetracycline base and the acid addition salts thereof,

(b) a magnesium compound selected from the group consisting of magnesium oxide, magnesium hydroxide, magnesium carbonate, magnesium acetate and magnesium chloride present in a molar ratio from 0.8 to 1.2 moles per mole of said antibiotic, with the proviso that magnesium chloride is used only with oxytetracycline base, and with the further proviso that when the magnesium compound is selected from magnesium chloride and magnesium acetate, then the antibiotic is present in an amount greater than 15 and up to 35 parts by weight.

(c) from 50 to 95 parts by weight of glycerol formal selected from the group consisting of 4-hydroxy-methyl-1,3-dioxolane, 5-hydroxy-1,3-dioxane and mixtures thereof,

(d) from 10 to 45 parts by weight of water,

(e) an antioxidant in an amount sufficient to stabilize said composition, and

(f) a buffering agent in an amount sufficient to provide a pH of from 6 to 9.5 in said composition.

2. The composition of claim 1 wherein said antibiotic is oxytetracycline base, said magnesium compound is magnesium oxide and said glycerol formal is a mixture of 4-hydroxymethyl-1,3-dioxolane and 5-hydroxy-1,3-dioxane.

3. The composition of claim 1 wherein said antibiotic is oxytetracycline hydrochloride, said magnesium compound is magnesium oxide and said glycerol formal is a mixture of 4-hydroxymethyl-1,3-dioxolane and 5-hydroxy-1,3-dioxane.

4. The composition of claim 2 wherein the mole ratio of said magnesium compound to said oxytetracycline base is 1 to 1.

5. The composition of claim 4 wherein the amount of oxytetracycline base present is 20% by weight of the total composition.

6. The composition of claim 3 wherein the mole ratio of said magnesium compound to said oxytetracycline hydrochloride is 0.8 to 1.

7. The composition of claim 1 wherein said antioxidant is sodium formaldehydesulfoxylate.

8. The composition of claim 1 wherein said buffering agent is monoethanolamine.

9. The composition of claim 1 wherein substantially all particles thereof are smaller than 0.20 μm.

10. In a vial, the antibiotic composition of claim 1 enveloped in an inert gas.

11. In a vial, the antibiotic composition of claim 5 enveloped in an inert gas.

## Claims for the Contracting State: AT

1. Process for the preparation of a stable, injectable antibiotic composition of increased antibiotic concentration for parenteral administration, characterized by mixing

(a) from 15 to 35 parts by weight of an antibiotic selected from the group consisting of oxytetracycline base and the acid addition salts thereof,

(b) a magnesium compound selected from the group consisting of magnesium oxide, magnesium hydroxide, magnesium carbonate, magnesium acetate and magnesium chloride in a molar ratio from 0.8 to 1.2 moles per mole of said antibiotic, with the proviso that magnesium chloride is used only with oxytetracycline base, and with the further proviso that when the magnesium compound is selected from magnesium chloride and magnesium acetate, then the antibiotic is present in an amount greater than 15 and up to 35 parts by weight,

(c) from 50 to 95 parts by weight of glycerol formal selected from the group consisting of 4-hydroxy-methyl-1,3-dioxolane, 5-hydroxy-1,3-dioxane and mixtures thereof,

(d) from 10 to 45 parts by weight of water,

(e) an antioxidant in an amount sufficient to stabilize said composition, and

(f) a buffering agent in an amount sufficient to provide a pH of from 6 to 9.5 in said composition.

2. Process of claim 1 wherein said antibiotic is oxytetracycline base, said magnesium compound is magnesium oxide and said glycerol formal is a mixture of 4-hydroxymethyl-1,3-dioxolane and 5-hydroxy-1,3-dioxane.

3. Process of claim 1 wherein said antibiotic is oxytetracycline hydrochloride, said magnesium compound is magnesium oxide and said glycerol formal is a mixture of 4-hydroxymethyl-1,3-dioxolane and 4-hydroxy-1,3-dioxane.

4. Process of claim 2 wherein the mole ratio of said magnesium compound to said oxytetracycline base is 1 to 1.

5. Process of claim 4 wherein the amount of oxytetracycline base present is 20% by weight of the total composition.

6. Process of claim 3 wherein the mole ratio of said magnesium compound to said oxytetracycline hydrochloride is 0.8 to 1.

7. Process of claim 1 wherein said antioxidant is sodium formaldehydesulfoxylate.

8. Process of claim 1 wherein said buffering agent is monoethanolamine.

9. Process of claim 1 wherein the composition is filtered through a 0.2 μm membrane filter.

10. Process according to any of the preceding claims wherein the antibiotic composition is filtered into a vial and enveloped in an inert gas.

**Patentansprüche für die Vertragsstaaten: BE CH DE FR GB IT LI LU NL SE**

1. Stabile, injizierbare antibiotische Zusammensetzung mit erhöhter Antibiotikum-Konzentration zur parenteralen Applikation, enthaltend:

(a) 15 bis 35 Gew.-Teile eines Antibiotikums ausgewählt aus der Gruppe bestehend aus Oxytetracyclinbase und den Säureadditionssalzen davon,

(b) eine Magnesiumverbindung, ausgewählt aus der Gruppe bestehend aus Magnesiumoxid, Magnesiumhydroxid, Magnesiumcarbonat, Magnesiumacetat und Magnesiumchlorid, die in einem Molverhältnis von 0,8 bis 1,2 Mol pro Mol des Antibiotikums vorhanden ist, mit der Bedingung, daß Magnesiumchlorid nur mit Oxytetracyclinbase verwendet wird, und der weiteren Bedingung, daß, wenn die Magnesiumverbindung ausgewählt ist aus Magnesiumchlorid und Magnesiumacetat, das Antibiotikum dann in einer Menge größer als 15 und bis zu 35 Gew.-Teilen vorhanden ist,

(c) 50 bis 95 Gew.-Teile GLycerinformal ausgewählt aus der Gruppe bestehend aus 4-Hydroxymethyl-1,3-dioxolan, 5-Hydroxy-1,3-dioxan und Mischungen davon,

(d) 10 bis 45 Gew.-Teile Wasser,

(e) ein Antioxidans in einer Menge, die ausreicht, um die Zusammensetzung zu stabilisieren, und

(f) eine Puffersubstanz in einer Menge, die ausreicht, um in der Zusammensetzung einen pH-Wert von 6 bis 9,5 zu ergeben.

2. Zusammensetzung nach Anspruch 1, dadurch gekennzeichnet, daß das Antibiotikum Oxytetracyclinbase ist, die Magnesiumverbindung Magnesiumoxid ist und das Glycerinformal eine Mischung aus 4-Hydroxymethyl-1,3-dioxolan und 5-Hydroxy-1,3-dioxan ist.

3. Zusammensetzung nach Anspruch 1, dadurch gekennzeichnet, daß das Antibiotikum Oxytetracyclinhydrochlorid ist, die Magnesiumverbindung Magnesiumoxid ist und das Glycerinformal eine Mischung aus 4-Hydroxymethyl-1,3-dioxolan und 5-Hydroxy-1,3-dioxan ist.

4. Zusammensetzung nach Anspruch 2, dadurch gekennzeichnet, daß das Molverhältnis der Magnesiumverbindung zur Oxytetracyclinbase 1:1 beträgt.

5. Zusammensetzung nach Anspruch 4, dadurch gekennzeichnet, daß die Menge an vorhandener Oxytetracyclinbase 20 Gew.-% der Gesamtzusammensetzung beträgt.

6. Zusammensetzung nach Anspruch 3, dadurch gekennzeichnet, daß das Molverhältnis der Magnesiumverbindung zum Oxytetracyclinhydrochlorid 0,8:1 beträgt.

7. Zusammensetzung nach Anspruch 1, dadurch gekennzeichnet, daß das Antioxidans Natriumformaldehydsulfoxylat ist.

8. Zusammensetzung nach Anspruch 1, dadurch gekennzeichnet, daß die Puffersubstanz Monoethanolamin ist.

9. Zusammensetzung nach Anspruch 1, dadurch gekennzeichnet, daß im wesentlichen alle Teilchen davon kleiner als 0,20 μm sind.

10. Die in einer Ampulle befindliche, von einem inerten Gas eingeschlossene antibiotische Zusammensetzung nach Anspruch 1.

11. Die in einer Ampulle befindliche von einem inerten Gas eingeschlossene antibiotische Zusammensetzung nach Anspruch 5.

**Patentansprüche für den Vertragsstaat: AT**

1. Verfahren zur Herstellung einer stabilen injizierbaren antibiotischen Zusammensetzung mit erhöhter Antibiotikum-Konzentration zur parenteralen Applikation, dadurch gekennzeichnet, daß man

(a) 15 bis 35 Gew.-Teile eines Antibiotikums ausgewählt aus der Gruppe bestehend aus Oxytetracyclinbase und den Säureadditionssalzen davon, mit

(b) einer Magnesiumverbindung, ausgewählt aus der Gruppe bestehend aus Magnesiumoxid, Magnesiumhydroxid, Magnesiumcarbonat, Magnesiumacetat und Magnesiumchlorid, die in einem Molverhältnis von 0,8 bis 1,2 Mol pro Mol des Antibiotikums eingesetzt wird, mit der Bedingung, daß Magnesiumchlorid nur mit Oxytetracyclinbase verwendet wird, und der weiteren Bedingung, daß, wenn die Magnesiumverbindung ausgewählt ist aus Magnesiumchlorid und Magnesiumacetat, das Antibiotikum dann in einer Menge größer als 15 und bis zu 35 Gew.-Teilen vorhanden ist,

(c) 50 bis 95 Gew.-Teile Glycerinformal ausgewählt aus der Gruppe bestehend aus 4-Hydroxymethyl-1,3-dioxolan, 5-Hydroxy-1,3-dioxan und Mischungen davon,

(d) 10 bis 45 Gew.-Teile Wasser,

(e) ein Antioxidans in einer Menge, die ausreicht, um die Zusammensetzung zu stabilisieren, und

(f) eine Puffersubstanz in einer Menge, die ausreicht, um in der Zusammensetzung einen pH-Wert von 6 bis 9,5 zu ergeben, vermischt.

2. Verfahren nach Anspruch 1, dadurch gekennzeichnet, daß das Antibiotikum Oxytetracyclinbase ist, die Magnesiumverbindung Magnesiumoxid ist und das Glycerinformal eine Mischung aus 4-Hydroxymethyl-1,3-dioxolan und 5-Hydroxy-1,3-dioxan ist.

3. Verfahren nach Anspruch 1, dadurch gekennzeichnet, daß das Antibiotikum Oxytetracyclinhydrochlorid ist, die Magnesiumverbindung Magnesiumoxid ist und das Glycerinformal eine Mischung aus 4-Hydroxymethyl-1,3-dioxolan und 5-Hydroxy-1,3-dioxan ist.

4. Verfahren nach Anspruch 2, dadurch gekennzeichnet, daß das Molverhältnis der Magnesiumverbindung zur Oxytetracyclinbase 1:1 beträgt.

5. Verfahren nach Anspruch 4, dadurch gekennzeichnet, daß die Menge an vorhandener Oxytetracyclinbase 20 Gew.-% der Gesamtzusammensetzung beträgt.

6. Verfahren nach Anspruch 3, dadurch gekennzeichnet, daß das Molverhältnis der Magnesiumverbindung zum Oxytetracyclinhydrochlorid 0,8:1 beträgt.

7. Verfahren nach Anspruch 1, dadurch gekennzeichnet, daß das Antioxidans Natriumformaldehydsulfoxylat ist.

8. Verfahren nach Anspruch 1, dadurch gekennzeichnet, daß die Puffersubstanz Monoäthanolamin ist.

9. Verfahren nach Anspruch 1, dadurch gekennzeichnet, daß man die erhaltene Zusammensetzung durch ein Membranfilter mit einer Porengröße von 0,2 µm filtriert.

10. Verfahren nach einem der vorangehenden Ansprüche, dadurch gekennzeichnet, daß man die Zusammensetzung in eine Ampulle unter Inertgas abfüllt.

**Revendications pour les Etats Contractants: BE CH DE FR GB IT LI LU NL SE**

1. Composition antibiotique stable injectable à concentration antibiotique accrue pour administration parentérale, caractérisée en ce qu'elle comprend:

a) de 15 à 35 parties en poids d'un antibiotique choisi dans le groupe constitué par la base oxytétracycline et les sels d'addition de cette base avec un acide.

b) un composé de magnésium choisi dans le groupe constitué par l'oxyde de magnésium l'hydroxyde de magnésium, le carbonate de magnésium, l'acétate de magnésium et le chlorure de magnésium, présent selon un rapport molaire de 0,8 à 1,2 moles par mole dudit antibiotique sous réserve que le chlorure de magnésium soit utilisé seulement avec la base oxytétracycline et sous la réserve supplémentaire que lorsque le composé de magnésium est choisi parmi le chlorure de magnésium et l'acétate de magnésium l'antibiotique soit alors présent en une proportion supérieure à 15 parties en poids et allant jusqu'à 35 parties en poids.

c) de 50 à 95 parties en poids de glycérol formal choisi dans le groupe constitué par le 4-hydroxy-méthyl-1,3-dioxolane, le 5-hydroxy-1,3-dioxane et leurs mélanges.

d) de 10 à 45 parties en poids d'eau.

e) un antioxydant en une quantité suffisante pour stabiliser ladite composition et

f) un agent tampon en une quantité suffisante pour fournir un pH de 6 à 9,5 dans ladite composition.

2. Composition selon la revendication 1, caractérisée en ce que ledit antibiotique est la base oxytétracycline, ledit composé de magnésium est 1 oxyde de magnésium et ledit glycérol formal est un mélange de 4-hydroxyméthyl-1,3-dioxolane et de 5-hydroxy-1,3-dioxane.

3. Composition selon la revendication 1, caractérisée en ce que ledit antibiotique est un chlorhydrate d'oxytétracycline, ledit composé de magnésium est de l'oxyde de magnésium et ledit glycérol formal est un mélange de 4-hydroxyméthyl-1,3-dioxolane et de 5-hydroxy-1,3-dioxane.

4. Composition selon la revendication 2, caractérisée en ce que le rapport molaire dudit composé de magnésium à ladite base oxytétracycline est de 1 à 1.

5. Composition selon la revendication 4, caractérisée en ce que la quantité de base oxytétracycline présente est de 20% poids par rapport au total de la composition.

6. Composition selon la revendication 3, caractérisée en ce que le rapport molaire dudit composé de magnésium audit chlorhydrate d'oxytétracycline est de 0,8 à 1.

7. Composition selon la revendication 1, caractérisée en ce que ledit anti-oxydant est le formaldéhyde-sulfoxylate de sodium.

8. Composition selon la revendication 1, caractérisée en ce que ledit agent tampon est la monoéthanolamine.

9. Composition selon la revendication 1, caractérisée en ce que pratiquement toutes ses particules sont inférieures à 0,20 microns.

10. Dans une fiole, la composition antibiotique selon la revendication 1 enveloppée dans un gaz inerte.

11. Dans une fiole, la composition antibiotique selon la revendication 5 enveloppée dans un gaz inerte.

**Revendications pour l'Etat Contractant: AT**

1. Procédé pour la préparation d'une composition antibiotique stable, injectable, à concentration antibiotique accrue, pour administration parentérale, caractérisée en ce que on combine

a) de 15 à 35 parties en poids d'un antibiotique choisi dans le groupe constitué par la base oxytétracycline et les sels d'addition de cette base avec un acide,

b) un composé de magnésium choisi dans le groupe constitué par l'oxyde de magnésium, l'hydroxyde de magnésium, le carbonate de magnésium, l'acétate de magnésium et le chlorure de magnésium, présent selon un rapport molaire de 0,8 à 1,2 moles par mole dudit antibiotique, sous réserve que le chlorure de magnésium soit utilisé seulement avec la base oxytétracycline et sous la réserve supplémentaire que lorsque le composé de magnésium est choisi parmi le chlorure de magnésium et l'acétate de magnésium, l'antibiotique soit alors présent en une proportion supérieure à 15 parties en poids et allant jusqu'à 35 parties en poids,

c) de 50 à 95 parties en poids de glycérol formal choisi dans le groupe constitué par le 4-hydroxy-méthyl-1,3-dioxolane, le 5-hydroxy-1,3-dioxane et leurs mélanges,

d) de 10 à 45 parties en poids d'eau,

e) un antioxydant en une quantité suffisante pour stabiliser ladite composition et,

f) un agent tampon en une quantité suffisante pour fournir un pH de 6 à 9,5 dans ladite composition.

2. Procédé selon la revendication 1, caractérisée en ce que ledit antibiotique est la base oxytétracycline, ledit composé de magnésium est 1 oxyde de magnésium et ledit glycérol formal est un mélange de 4-hydroxyméthyl-1,3-dioxolane et de 5-hydroxy-1,3-dioxane.

3. Procédé selon la revendication 1, caractérisée en ce que ledit antibiotique est un chlorhydrate d'oxytétracycline, ledit composé de magnésium est de l'oxyde de magnésium et ledit glycérol formal est un mélange de 4-hydroxyméthyl-1,3-dioxolane et de 5-hydroxy-1,3-dioxane.

4. Procédé selon la revendication 2, caractérisée en ce que le rapport molaire dudit composé de magnésium à ladite base oxytétracycline est de 1 à 1.

5. Procédé selon la revendication 4, caractérisée en ce que la quantité de base oxytétracycline présente est de 20% poids par rapport au total de la composition.

6. Procédé selon la revendication 3, caractérisée en ce que le rapport molaire dudit composé de magnésium audit chlorhydrate d'oxytétracycline est de 0,8 à 1.

7. Procédé selon la revendication 1, caractérisée en ce que ledit anti-oxydant est le formaldéhyde-sulfoxylate de sodium.

8. Procédé selon la revendication 1, caractérisée en ce que ledit agent tampon est la monoéthanolamine.

9. Procédé selon la revendication 1, caractérisée en ce que on filtre la composition par un filtre à 0,20 µm.

10. Procédé selon une des revendications précédentes, caractérisée en ce que on remplit la composition, enveloppée dans un gaz inerte, dans une fiole.